(19) Europäisches Patentamt
European Patent Office
Office européen des brevets

(11) Publication number: **0 169 614**
**B1**

# (12) EUROPEAN PATENT SPECIFICATION

(45) Date of publication of patent specification: 30.11.88

(51) Int. Cl.[4]: C 07 C 101/30

(21) Application number: 85201165.9

(22) Date of filing: 10.07.85

## (54) Process for the preparation of L-carnitine.

(30) Priority: 12.07.84 IT 2185884

(43) Date of publication of application:
29.01.86 Bulletin 86/05

(45) Publication of the grant of the patent:
30.11.88 Bulletin 88/48

(84) Designated Contracting States:
AT BE CH DE FR GB LI LU NL SE

(56) References cited:
GB-A-2 132 614
ACTA CHEMICA SCANDINAVICA, vol. B-37, 1983, pages 340-344, Copenhagen, DK; K. BOCK et al.: "Synthesis of S-and R-4-amino-3-hydroxybutyric acid (GABOB) and S- and R-carnitine from arabinose or ascorbic acid"
TETRAHEDRON LETTERS, vol. 26, no. 1, 1985, pages 101-104, Pergamon Press Ltd., GB; C. FUGANTI et al.: "On the steric course of baker's yeast mediated reduction of alkyl 4-azido- and 4-bromo-3-oxobutyrate. Synthesis of (R)- and (S)-carnitin"

(73) Proprietor: SCLAVO S.p.A.
Via Fiorentina 1
I-53100 Siena (IT)
(73) Proprietor: CONSIGLIO NAZIONALE DELLE RICERCHE
Viale Liegi 48/B
I-00198 Roma (IT)

(72) Inventor: Casati, Paolo
Via della Birona 27
I-20052 Monza Milan (IT)
Inventor: Carmeno, Massimo
Via Ticino 18
I-20097 S. Donato Milanese Milan (IT)
Inventor: Benedetti, Alberto
Residenza Fontana
I-20097 Segrate Milan (IT)
Inventor: Fuganti, Claudio
Via G.B. Nazzari 8
I-20129 Milan (IT)
Inventor: Grasselli, Piero
Via T. Calzecchi 10
I-20133 Milan (IT)

(74) Representative: Appoloni, Romano et al
ING. BARZANO' & ZANARDO MILANO S.p.A. Via Borgonuovo 10
I-20121 Milano (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 169 614 B1

Courier Press, Leamington Spa, England.

**Description**

The present invention relates to a process for the synthesis of L-carnitine

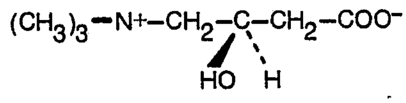

(I)

Compound (I), also known as vitamin $B_T$, is a hydroxyaminoacid stimulating the mitochondrial oxidation of fatty acids, productin energy at muscular level.

Its most known use is as a tonic, although it has been recently used in other therapeutical fields, such as the chronic hemodialysis, the correction of lipoproteinic pattern, the suppression of cardiac arrhytmia during the course of infarct, and the infusional therapy.

Compound (I) can be isolated by known processes from natural substrates, such as meat extract, with an end yield of 2—1%, that renders the same process economicaly disadvantageous, and however not much suitable on a commercial scale.

According to Carter and Bhattacharyye [J. Am. Chem. Soc. 75, 2503 (1953)] D,L-carnitine is obtained by chemical synthesis from benzaldehyde and epichlorohydrin through a plurality of steps, with an overall yield of 20—25%.

According to U.S. Pat. 3,135,788 D,L-carnitine chloride salt is prepared from epihalogenohydrin and trimethylamine through a plurality of steps, with the production of 3-halogeno-2-hydroxypropyl trimethylammonium chloride, which is then reacted with potassium or sodium cyanide to yield a 3-cyano-2-hydroxypropyl trimethylammonium chloride. The compound so obtained is then hydrolized to produce D,L-carnitine.

According to said known processes, the chemical synthesis of carnitine is carried out starting from non-optically-active compounds, or racemic mixtures, with the attendant formation of both the "L" and "D" isomers of carnitine.

In the last steps of such processes, the separation is then carried out of the L-isomer from the reaction mixture by fractional crystallization, using optically-active acids: such an approach involves a maximum yield of 50% and thus the entire procedure is not economically advatageous.

To redress this shortcoming, Bock et al., ACTA CHEMICAL SCANDINAVICA, Vol. *B—37,* (1983), pages 340—344, Copenhagen, DK, dscribe the exploitation of L(+)arabinose as the starting substrate for synthesizing L-carnitine: thus, L(+)arabinose is oxidized to L-erythronate, whereafter, by reaction with HBr in acetic acid and methanol, one obtains the compound methyl-2,4-dibromo-2, 4-dideoxy-L-erythronate, to be subjected to selective hydrogenolysis in the 2-position: in the product thus obtained, the trimethylamine group is then introduced and saponification is carried out.

In the latter process, therefore, there is already present, in the starting compound, and in the suitable position, a carbon atom having the appropriate configuration, which is never involved in the entire synthesizing sequence.

In the technical and patent literature, processes for the microbiological synthesis of L-carnitine have recently been described.

According to GB—A—2 078 742, the synthesis of L-carnitine is carried out by means of the hydroxylation of gamma-butyrine with *Neurospora crassa.*

According to FR—A—2 398 046, L-carnitine is obtained by means of the oxidation of D,L-carnitine to the corresponding 3-keto derivative, and subsequent stereospecific reduction to L-carnitine, using enzymic systems induced in *Pseudomonas.*

B. Zhan et al describe, in J. Am. Chem. Soc. 1983, *105,* 5925—5926, a method for synthesizing L-carnitine by means of the microbiological stereospecific reduction of the esters of 4-chloro-3-ketobutyric acid and esters of 4-cloro-3-hydroxybutyric acid of (3*R*) configuration: the product thus obtained is then treated with trimethylamine and ethanol at 80°C, and by subsequent acidic hydrolysis.

Also in GB—A—2 132 614, L-carnitine is synthesized by using, as the procedure for obtaining the chiral centre, the reduction of a carbonyl precursor by a microorganism capable of synthesizing oxyreductive enzymes, and, more particularly, the substrate is an ester of the 4-halo-3-ketobutyric acid with a straight-line alcohol having from 1 to 10 C-atoms, or an amide of such acid with a lipophilic amine.

It is, however, known (B. Zhan et al., loc. cit.), that, for alkoxy radicals having less than 6 carbon atoms the stereochemistry of the reduction reaction is reversed and the S-enantiomer is predominantly obtained: the latter has no more a configuration suitable for being used in L-carnitine synthesis.

Although the latter microbiological approaches aimed directly to producing the L-isomer, they have proven cumbersome, difficult to carry out, expensive and giving not satisfactory yields.

The principal objective of this invention is, therefore, to provide a process which is not jeopardized by the defects discussed in the foregoing.

The invention therefore, provides a process for synthesizing L-carnitine:

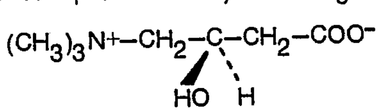

(I)

characterized in that it comprises the steps of:

a) contacting an ester of the 4-azido-3-ketobutyric acid:

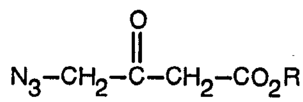

(II)

wherein R is either $C_2$—$C_{10}$ alkyl radical, or a $C_7$—$C_{10}$ alkylaryl radical, in the liquid phase and in the presence of both a carbon source and a phosphate buffer, with a micro-organism capable of generating oxyreductive enzymes which is the Saccharomyces cerevisiae, to give an ester of the 4-azido-3-hydroxybutyric acid:

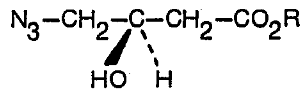

(III)

in the form of the (3*R*) isomer thereof;

b) subjecting the compound (III) to the hydrolysis of its ester group in the presence of a base, to give the (3*R*)-4-azido-3-hydroxybutyric acid:

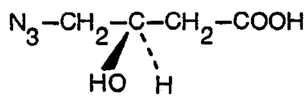

(IV)

c) hydrogenating the azido group of the compound (IV) with gaseous hydrogen in the presence of a hydrogenation catalyst, to give (3*R*)-4-amino-3-hydroxybutyric acid [(−)-GABOB]:

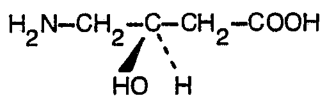

(V)

and

d) methylating the compound (V) to give L-carnitine (I).

The weight ratio of the microorganisms to the compound (II) is comprised between 1:50 and 1:5, the enzymatic reaction being carried out in an aqueous medium, in the presence of a carbon source and a phosphate buffer, such as Na-,K-,$NH_4$-phosphate at a pH of from 5 to 10. The reaction temperature is from 10°C to 40°C and the reaction time varies from 1 hour to 6 hours.

The *Saccharomyces cerevisiae,* using glucose as a nutrient, the pH being from 6,5 to 7,5, the preferred temperature being 37°C and the reaction time being 3 hours.

On completion of the reaction, the sediment is collected on a filter, or centrifuged and washed with water, the liquid phases being combined and extracted with a solvent such as ethyl acetate, $CH_2Cl_2$, $CHCl_3$ and hexane.

The solvent is evaporated off and the residue is thus recovered.

At the NMR analysis with chiral shift reactants, said residue results to be constituted by (3*R*) enantiomer of the ester of 4-azido-3-hydroxybutyric acid [compound (III)].

In the step (b) of the present invention, the compound (III) is hydrolyzed at its ester group, by means of one of known techniques.

The reaction is generally carried out in the liquid phase with a base selected among sodium, potassium or calcium hydroxide at room temperatures (20—25°C); and for a time of from 1 to 5 hours. At the end of such a time period, the alcohol formed during the hydrolysis reaction is separated and recovered.

The aqueous solution is then made acid and the residue is recovered by extraction with an organic solvent selected among ethyl acetate, $CH_2Cl_2$, $CHCl_3$, hexane.

In the step (c) of the present invention, the 4-azido-3-hydroxybutyric acid (IV) so obtained from the preceding step is hydrogenated at its azido group with hydrogen gas and in the presence of a hydrogenation catalyst, to yield the compound (V), 4-amino-3-hydroxybutyric acid, also named (−)-GABOB, useful as an antiepileptic and hypotensive agent.

The hydrogenation catalysts used to that purpose may be constituted by any hydrogenation catalysts known from the art, e.g., catalysts of noble metals.

In the preferred embodiment however, such catalysts as Pd/C or $PtO_2$ are used.

The amount of catalysts used in the hydrogenation of the compound (IV) is generally such as to have a weight ratio of compound (IV)/catalyst of from 100/1 to 20000/1.

The hydrogenation of compound (IV) is then carried out in the liquid phase, with the catalyst either suspended or dissolved depending of the particular catalyst used, in an inert organic solvent.

Solvents suitable to that purpose are hydrocarbons, alcohols, ethers and esters or organic acids, liquid under the conditions under which the hydrogenation reaction is carried out.

Specific examples of solvents belonging to the above indicated classes are acetic acid and ethanol.

The temperatures at which the hydrogenation reaction is carried out may generally vary within the range of from 10°C to 60°C, and the corresponding reaction times range generally from 20 hours to 20 minutes.

The reaction is typically carried out at room temperatures (20—25°C) and in this case the time required to complete or to essentially complete the reaction is of about 4 hours.

The hydrogenation reaction may be carried out under a hydrogen pressure equal to or higher than the atmospheric pressure and in general it is not convenient to exceed a value of about 50 atmospheres.

By operating under the previously indicated conditions compound (V) is obtained in a yield of 95% relatively to the starting substrate.

At the end of the reaction, the catalyst is removed by extraction, filtering or by other suitable techniques, and the solvent is removed, generally by evaporation.

The compound (V) is then recovered from the reaction mixture by crystallization from water, from alcohol or from water-alcohol mixtures.

In the step (e) compound (V) is transformed into L-carnitine by methylation.

In this step, any known technique may be used. In practice, the reaction is carried out by using a solution of methyl iodide in methanol and potassium hydroxide. After a reaction time of 36 hours at a temperature of 60°C, the solvent is evaporated to dryness and the residue is extracted four times with a phenol/water solution.

The combined extracts are placed in a separation funnel containing diethyl ether, and the phenol/diethyl ether layer is separated from water, and washed with water.

The combined aqueous extracts are eluted through a column containing an ion-exchange resin, and the eluate is evaporated to dryness under reduced pressure.

By this process, crystals of L-carnitine are obtained with a yield of 80% and with an optical purity of 95—100%.

The compound (II) of the present invention may be prepared by means of known processes, by starting from the corresponding ester of 4-chloro-3-ketobutyric acid or of 4-bromo-3-ketobutyric acid, and sodium azide.

The reaction is typically carried out by placing in contact the said compounds in a water-alcohol mixture, at a temperature of about 70°C, and for a time of the order of three hours.

The organic solvent is then evaporated off and the compound (II) is recovered from the reaction mixture.

The following experimental Examples are illustrative and not limitative of the invention.

Example 1

Preparation of the ester of 4-azido-3-ketobutyric acid

Into a glass flask of 3 l in volume, equipped with stirring means, 248 g (1 mol) of octyl ester of 4-chloro-3-ketobutyric acid, 715 g (1.1 mol) of sodium azide and 1500 ml of a water-methanol (1:1) mixture are loaded. The solution is maintained at a temperature of 70°C for a time of about 3 hours.

At the end of said time period, 1000 ml of water are added and the whole mixture is subsequently extracted 3 times with 1000 ml of organic solvent, such as ethyl acetate or $CH_2Cl_2$.

The solvent is evaporated to dryness and 220 g are obtained of the compound (II) ($R = C_8H_{17}$), with a purity, as analyzed by NMR, of 95%.

Example 2

Preparation of the ester of 4-azido-3-hydroxybutyric acid

Into a glass flask, of 3 l in capacity, provided with stirring means, 1 l of an aqueous solution containing 100 g of D-glucose and 10 g of $Na_2HPO_4$, and 250 g of bread-making yeast (Saccharomyces cerevisiae) are loaded. To the said suspension, kept stirred, 8.0 g of the compound (II) are added over a time period of about 10 minutes, with the temperature being controlled at values lower than 37°C. At the end of such time, the suspension is maintained at a temperature of 37°C for about 3 hours.

Said suspension is then extracted twice with 500 ml of solvent, either ethyl acetate or $CH_2Cl_2$. At the end, the solvent is evaporated to dryness.

An amount of 7.5 g is obtained of the octyl ester of 4-azido-3-hydroxybutyric acid [compound (III)]. On NMR analysis with chiral shift reactants, said compound results to be constituted by the (3*R*) enantiomer only.

Example 3

Preparation of 4-azido-3-hydroxybutyric acid

Into a glass flask, of 1 l in capacity, 25.5 of the octyl ester of 4-azido-3-hydroxybutyric acid as obtained as disclosed in Example 2; 10 g of potassium hydroxide, 500 ml of water and 200 ml of methanol are loaded. The reaction mixture is boiled over two hours, is concentrated in vacuo to remove methanol, is cooled and octyl alcohol is extracted with 200 ml of $CH_2Cl_2$. The aqueous solution is made acid to pH 1 with 6N hydrocloric acid and is extracted twice with 200 ml of $CH_2Cl_2$. The residue obtained by evaporating the solvent is constituted by acid (IV), in an amount of 13.4 g (90%).

### Example 4

Preparation of 4-amino-3-hydroxybutyric acid [(−)-GABOB]

Into a hydrogenation reactor of 0.5 l in capacity, provided with stirring means, 14.6 g (0.1 mol) of the compound (IV), as prepared according to the preceding Example 3; 150 ml of glacial acetic acid and 500 mg of $PtO_2$ are charged, and the reaction is carried out under a hydrogen pressure of 4 atmospheres over three hours.

At the end of this time period, the mixture is evaporated to dryness, the residue is collected with water and is filtered to remove the catalyst.

The filtrate is crystallized from water-alcohol to separate 4-amino-3-hydroxybutyric acid (V).

An amount of 11.9 g is obtained (yield 100%). The compound has $[\alpha]_d^{20°} = -21$ (Cl, $H_2O$), thus resulting enantiomorphically pure.

### Example 5

Preparation of L-carnitine

A solution of 5.8 g of methyl iodide in 60 ml of methanol is added to a solution of 1.2 g of compound (V) and 2.2 g of potassium hydroxide in 10 ml of water.

The solution is maintained at the temperature of 60°C overnight. At the end, the solution is evaporated to dryness, and the residue is collected with 50 ml of water.

The aqueous solution is extracted 4 times with 50 ml of 80%-phenol.

The combined phenolic extracts are washed with 20 ml of water, and are then added to 600 ml of diethyl ether.

In a separation funnel, the aqueous layer is separated from the ether-phenol layer, and is subsequently extracted four times with 50 ml of water. The combined aqueous extracts are washed with 150 ml of diethyl ether and are eluted through a column containing an anionic resin.

The alkaline fraction of the eluate is then concentrated in vacuo up to dryness. The residue is filtered by using an ethanol-acetone (2:3) solution, and 0.97 g (80%) of L-carnitine are obtained.

The compound has $[\alpha]_d^{20°} = -30$ (Cl, $H_2O$), thus resulting enantiomorphically pure.

### Example 6

Preparation of (3*R*)-3-hydroxy-4-azidobutyric acid, key intermediate in the synthesis of R-carnitin from ethyl-, benzyl-, and phenethyl 4-azido-3-oxobutyrates and microbial reduction

Ethyl-, benzyl-, and phenethyl 4-azido-3-oxobutyrates were prepared from the corresponding 4-chloro derivatives by treatment with sodium azide, using exactly the same dilutions and the same molar ratio and the same experimental conditions described for the preparation of the octyl ester, obtaining nearly the same yields.

The yeast reduction of ethyl-, benzyl-, and phenethyl 4-azido-3-oxobutyrate to 4-azido-3-hydroxy esters was carried on as reported for the octyl ester, using exactly the same molar concentrations. The reduced materials were isolated as reported, in the same yields.

By means of $^1H$ NMR studies with chiral shift reagents, the ethyl 4-azido-3-hydroxybutyrate was shown to contain ca 90% of the (3*R*) enantiomer. Similarly, the benzyl- and the phenethyl derivatives were shown to contain over 95% of the (3*R*) enantiomers.

The conversion of the ethyl-, benzyl-, and phenethyl 4-azido-3-hydroxybutyrates into 4-azido-3-hydroxybutyric acid was performed by basic treatment under conditions identical in terms of dilutions and molar ratio and experimental conditions as reported for the octyl ester, obtaining the same yields.

## Claims

1. Process for synthesizing L-carnitine:

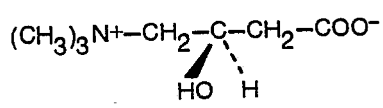

(I)

characterized in that it comprises the steps of:

a) contacting an ester of the 4-azido-3-ketobutyric acid:

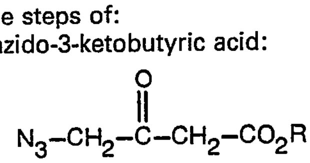

(II)

wherein R is either $C_2$—$C_{10}$ alkyl radical, or a $C_7$—$C_{10}$ alkylaryl radical, in the liquid phase and in the presence of both a carbon source and a phosphate buffer, with a micro-organism which is the Saccharomyces cerevisiae, to give an ester of the 4-azido-3-hydroxybutyric acid:

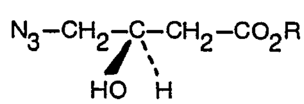

(III)

in the form of the (3*R*) isomer thereof;

b) subjecting the compound (III) to the hydrolysis of its ester group in the presence of a base, to give the (3*R*)-4-azido-3-hydroxybutyric acid:

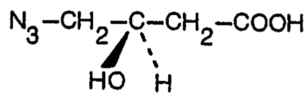

(IV)

c) hydrogenating the azido group of the compound (IV) with gaseous hydrogen in the presence of a hydrogenation catalyst, to give (3*R*)-4-amino-3-hydroxybutyric acid [(−)-GABOB]:

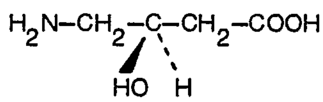

(V)

and

d) methylating the compound (V) to give L-carnitine (I).

2. Process according to claim 1, wherein the step (a) is carried out at a pH of from 5 to 10, at a temperature of from 10°C to 40°C and for a time of from 1 to 6 hours.

3. Process according to claim 1, wherein the carbon source is D-glucose.

4. Process according to claim 1, wherein the step (a) is carried out at a pH of from 6,5 to 7,5, at the temperature of 37°C and for a time of 3 hours.

5. Process according to claim 1, wherein the base to be used in the step (b) is selected from among sodium hydroxide, potassium hydroxide and calcium hydroxide.

6. Process according to claim 1, wherein the step (b) is carried out at a temperature of from room temperature to the boiling point temperature of the liquid phase and for a time of from 1 to 5 hours.

7. Process according to claim 1, wherein the step (c) the hydrogenation catalyst is $PtO_2$.

8. Process according to claim 1, wherein step (c) is carried out in an inert solvent medium, at a temperature of from 10°C to 60°C, for a time of from 20 minutes to 20 hours and under a partial hydrogen pressure of from 98,066 kPa to 4903,3 kPa.

9. Process according to claim 8, wherein said inert solvent is a liquid selected from among hydrocarbons, alcohols, ethers, esters and organic acids.

10. Process according to claim 9, wherein the solvent medium is either or acetic acid.

11. Process according to claim 8, wherein the step (c) is carried out at a temperature between 20°C and 25°C and for a time of at least 4 hours.

12. Process according to claim 1, wherein step (d) is carried out using a methanol solution of methyl iodide and an alkali metal compound as the methylating medium.

**Patentansprüche**

1. Verfahren zur Synthese von L-Carnitin:

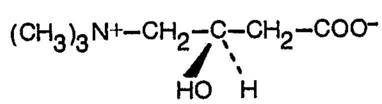

(I)

dadurch gekennzeichnet, daß es die folgenden Stufen umfaßt:

a) In-Berührung-Bringen eines Esters der 4-Azido-3-ketobuttersäure:

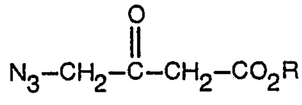

(II)

worin R entweder einen $C_2$—$_{10}$Alkylrest oder einen $C_7$—$C_{10}$Alkylarylrest bedeutet, in flüssiger Phase und in Gegenwart von sowohl einer Kohlenstoffquelle als auch eines Phosphatpuffers mit einem Mikroorganismus, der Saccharomyces cerevisiae ist, zur Ausbildung eines Esters der 4-Azido-3-hydroxybuttersäure:

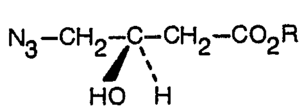

(III)

in Form des (3*R*)-Isomers hievon;

b) Ausführung einer Hydrolyse der Estergruppe der Verbindung (III) in Gegenwart einer Base zur Ausbildung der (3*R*)-4-Azido-3-hydroxybuttersäure:

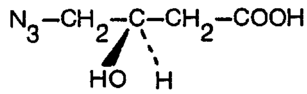

(IV)

c) Hydrieren der Azidogruppe der Verbindung (IV) mit gasförmigem Wasserstoff in Gegenwart eines Hydrierkatalysators unter Ausbildung der (3*R*)-4-Amino-3-hydroxybuttersäure [(−)-GABOB]:

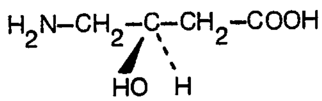

(V)

und

d) Methylieren der Verbindung (V) zu L-Carnitin (I).

2. Verfahren nach Anspruch 1, worin die Stufe (a) bei einem pH-Wert von 5 bis 10, bei einer Temperatur von 10 bis 40°C und während einer Zeitdauer von 1 bis 6 Stunden ausgeführt wird.

3. Verfahren nach Anspruch 1, worin die Kohlenstoffquelle D-Glucose ist.

4. Verfahren nach Anspruch 1, worin die Stufe (a) bei einem pH-Wert von 6,5 bis 7,5 bei einer Temperatur von 37°C und während einer Zeitdauer von 3 Stunden ausgeführt wird.

5. Verfahren nach Anspruch 1, worin die in der Stufe (b) einzusetzende Base unter Natriumhydroxid, Kaliumhydroxid und Kalziumhydroxid ausgewählt wird.

6. Verfahren nach Anspruch 1, worin die Stufe (b) bei einer Temperatur von Raumtemperatur bis zum Siedepunkt der flüssigen Phase und während einer Zeit von 1 bis 5 Stunden ausgeführt wird.

7. Verfahren nach Anspruch 1, worin in Stufe (c) der Hydrierkatalysator $PtO_2$ ist.

8. Verfahren nach Anspruch 1, worin die Stufe (c) in einem inerten Lösungsmittel bei einer Temperatur von 10 bis 60°C während einer Zeitdauer von 20 Minuten bis 20 Stunden und unter einem Wasserstoffpartialdruck von 98,066 kPa bis 4903,3 kPa ausgeführt wird.

9. Verfahren nach Anspruch 8, worin das inerte Lösungsmittel eine unter Kohlenwasserstoffen, Alkoholen, Ethern, Estern und organischen Säuren ausgewählt Flüssigkeit ist.

10. Verfahren nach Anspruch 9, worin das Lösungsmittelmedium entweder Ethanol oder Essigsäure ist.

11. Verfahren nach Anspruch 8, worin die Stufe (c) bei einer Temperatur zwischen 20°C und 25°C und während einer Zeitdauer von wenigstens 4 Stunden ausgeführt wird.

12. Verfahren nach Anspruch 1, worin die Stufe (d) unter Verwendung einer Methanollösung von Methyliodid und einer Alkalimetallverbindung als Methylierungsmedium ausgeführt wird.

**Revendications**

1. Procédé pour la synthèse de la L-carnitine:

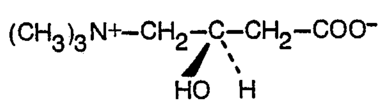

(I)

caractérisé en ce qu'il comprend les étapes de:

a) mise en contact d'un ester de l'acide 4-azido-3-cétobutyrique:

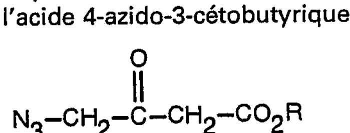

(II)

dans lequel R est soit un radical alkyle en $C_2$—$C_{10}$, soit un radical alkylaryle en $C_7$—$C_{10}$, en phase liquide et en présence à la fois d'une source de carbone et d'un tampon phosphate, avec un micro-organisme qui est *Saccharomyces cerevisiae,* pour obtenir un ester de l'acide 4-azido-3-hydroxybutyrique:

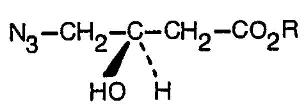

(III)

sous forme de l'isomère (3*R*) de celui-ci;

b) exposition du composé (III) à l'hydrolyse de son groupe ester en présence d'une base, pour obtenir l'acide (3*R*)-4-azido-3-hydroxybutyrique:

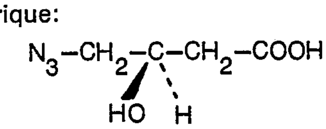

(IV)

c) hydrogénation du groupe azido du composé (IV) avec de l'hydrogène gazeux, en présence d'un catalyseur d'hydrogénation, pour obtenir l'acide (3*R*)-4-amino-3-hydroxybutyrique [(−)-GABOB]:

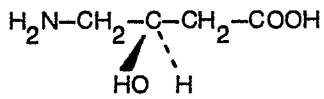

(V)

et

d) méthylation du composé (V) pour obtenir la L-carnitine (I).

2. Procédé selon la revendication 1, dans lequel l'étape (a) est effectuée à un pH de 5 à 10, à une température de 10 à 40°C, et pendant une durée de 1 à 6 heures.

3. Procédé selon la revendication 1, dans lequel la source de carbone est le D-glucose.

4. Procédé selon la revendication 1, dans lequel l'étape (a) est effectuée à un pH de 6,5 à 7,5, à la température de 37°C, et pendant une durée de 3 heures.

5. Procédé selon la revendication 1, dans lequel la base à utiliser dans l'étape (b) est choisie parmi l'hydroxyde de sodium, l'hydroxyde de potassium et l'hydroxyde de calcium.

6. Procédé selon la revendication 1, dans lequel l'étape (b) est effectuée à une température comprise entre la température ambiante et la température du point d'ébullition de la phase liquide, et pendant une durée de 1 à 5 heures.

7. Procédé selon la revendication 1, dans lequel le catalyseur d'hydrogénation utilisé dans l'étape (c) est $PtO_2$.

8. Procédé selon la revendication 1, dans lequel l'étape (c) est effectuée dans un milieu constitué d'un solvant inerte, à une température de 10 à 60°C, pendant une durée de 20 minutes à 20 heures, et sous une pression partielle d'hydrogène de 98,066 kPa à 4 903,3 kPa.

9. Procédé selon la revendication 8, dans lequel ledit solvant inerte est un liquide choisi parmi des hydrocarbures, des alcools, des éthers, des esters et des acides organiques.

10. Procédé selon la revendication 9, dans lequel le solvant est soit l'éthanol, soit l'acide acétique.

11. Procédé selon la revendication 8, dans lequel l'étape (c) est effectuée à une température comprise entre 20 et 25°C, pendant une durée d'au moins 4 heures.

12. Procédé selon la revendication 1, dans lequel on effectue l'étape (d) en utilisant, en tant que l'agent de méthylation, une solution d'iodure de méthyle dans du méthanol et un composé d'un métal alcalin.